# EUROPÄISCHE PATENTSCHRIFT

(11) **EP 1 675 811 B1**
(45) Veröffentlichungstag und Bekanntmachung des Hinweises auf die Patenterteilung: **23.01.2008**
(21) Anmeldenummer: 04790431.3
(22) Anmeldetag: 14.10.2004
(51) Int. Cl.: C07C 43/11, C11D 1/825

(54) **C10-ALKANOLALKOXYLAT-GEMISCHE UND IHRE VERWENDUNG ALS NEUE SCHAUMARME NETZER**
C10 ALKANOLALKOXYLATE MIXTURES AND USE THEREOF AS NOVEL LOW-FOAMING WETTING AGENTS
MELANGES D'ALCANOLALCOXYLATS C10 ET LEUR UTILISATION, COMME NOUVEAUX AGENTS MOUILLANTS FAIBLEMENT MOUSSANTS

(30) Priorität: 14.10.2003 DE 10348420
(43) Veröffentlichungstag der Anmeldung: 05.07.2006
(73) Patentinhaber: BASF Aktiengesellschaft, 67056 Ludwigshafen (DE)
(72) Erfinder: KLUMPE, Markus, 68309 Mannheim (DE); TROPSCH, Jürgen, 67354 Römerberg (DE); KAHL, Rolf-Dieter, 67454 Hassloch (DE); BÖHN, Roland, 67136 Fussgönheim (DE); STUTZ, Susanne, Weinheim 69469 (DE); NÖRENBERG, Ralf, Ingelheim 55218 (DE)
(74) Vertreter: Isenbruck, Günter
(86) Internationale Anmeldenummer: PCT/EP2004/011575
(87) Internationale Veröffentlichungsnummer: WO 2005/037757

(56) Entgegenhaltungen:
- EP-A- 0 667 893
- WO-A-00/74845
- WO-A-94/11330
- WO-A-03/091190

## Beschreibung

Die vorliegende Erfindung betrifft die Verwendung von C₁₀-Alkanolalkoxylat-Gemischen, derartige C₁₀-Alkanolalkoxylat-Gemische und Verfahren zu ihrer Herstellung.

Alkoxylate von aliphatischen Alkoholen werden in großem Umfang als Tenside, Emulgatoren oder Schaumdämpfer eingesetzt. Die Benetzungs- und Emulgatoreigenschaften hängen dabei stark von der Art des Alkohols und der Art und Menge der Alkoxid-Addukte ab.

WO 94/11331 betrifft die Verwendung von Alkoxylaten von 2-Propylheptanol in Detergenzzusammensetzungen zur Entfettung harter Oberflächen. Die Alkoxylate weisen 2 bis 16 Alkylenoxid-Gruppen auf. Vorzugsweise liegt der überwiegende Teil der Alkylenoxid-Gruppen in Form von Ethylenoxid vor. Gemäß der Beispiele werden ausschließlich ethoxylierte Alkohole eingesetzt. Es ist ferner beschrieben, dass die Alkohole zunächst mit Ethylenoxid und sodann mit Propylenoxid umgesetzt werden können. Für derartige Alkoxylate sind jedoch keine Beispiele oder Eigenschaften angegeben. Es wird ausgeführt, dass die beschriebenen Alkoxylate eine gute Detergenz- und Benetzungswirkung zeigen, verbunden mit einem geringen Schäumen. Zudem wird angegeben, dass die Alkoxylate einen erwünschten Verdickungseffekt in Formulierungen haben.

WO 94/11330 betrifft Alkoxylate von 2-Propylheptanol und deren Verwendung. In den Alkoxylaten liegt 2-Propylheptanol, zunächst mit 1 bis 6 mol Propylenoxid und sodann mit 1 bis 10 mol Ethylenoxid umgesetzt, vor. Gemäß den Beispielen wird ein zunächst mit 4 mol Propylenoxid und sodann mit 6 mol Ethylenoxid umgesetztes 2-Propylheptanol eingesetzt. Es wird angegeben, dass die Alkylenoxidaddukte ein verbessertes Verhältnis von Schaumverhalten zu Detergenzwirkung zeigen. Ferner ist angegeben, dass die Alkoxylate ein gutes Benetzungsverhalten zeigen. Sie werden in Detergenzzusammensetzungen zur Reinigung von Textilmaterialien eingesetzt.

US 2,508,036 betrifft die Verwendung von 2-n-Propylheptanolethoxilaten, die 5 bis 15 mol Ethylenoxid enthalten, als Netzmittel in wässrigen Lösungen. Es ist beschrieben, dass die Produkte als Tenside in Waschmitteln eingesetzt werden können. Verfahren zur Alkoxylierung von 2-Propylheptanol sind prinzipiell aus dem Stand der Technik bekannt. In der WO 01/04183 wird beispielsweise ein Verfahren zur Ethoxylierung von hydroxyfunktionellen Starterverbindungen beschrieben, das in Gegenwart einer Doppelmetallcyanid-Verbindung als Katalysator durchgeführt wird.

Die prioritätsälteren, nicht vorveröffentlichten DE-A-102 18 753 und DE-A-102 43 363 betreffen Alkoxylat-Gemische, die sich insbesondere von 2-Propylheptanol durch Alkoxylierung ableiten. Dabei wird entweder ausschließlich mit Ethylenoxid oder Propylenoxid alkoxyliert, oder es wird zunächst mit Propylenoxid und sodann mit Ethylenoxid alkoxyliert. Es wird angegeben, dass auch zunächst Ethylenoxideinheiten und daran anschließend Propylenoxid-Einheiten vorliegen können. Neben statistischen Gemischen von Ethylenoxid-Einheiten und Propylenoxid-Einheiten werden auch eine 3- oder Mehrblockalkoxylierung und gemischte Alkoxylierung als Möglichkeiten aufgeführt.

Aufgabe der vorliegenden Erfindung ist die Bereitstellung von. Alkanolalkoxylaten, die als Emulgator, Schaumregulierer und als Netzmittel für harte Oberflächen geeignet sind. Die Alkoxylate sollen insbesondere ein gutes Emulgierverhalten und einen geringen Kontaktwinkel auf harten Oberflächen bei der Anwendung zeigen. Ferner sollen sie die Grenzflächenspannung in flüssigen Systemen vermindern. Die Alkoxylate sollen allgemein ein vorteilhaftes Eigenschaftsspektrum bei der Verwendung als Emulgator, Schaumregulierer oder als Netzmittel zeigen. Des weiteren sollte die Menge an Restalkohol reduziert werden, um Geruchsbeeinträchtigungen zu vermeiden.

Die Aufgabe wird erfindungsgemäß gelöst durch Alkoxylat-Gemische, enthaltend Alkoxylate der allgemeinen Formel (1)

C₅H₁₁CH(C₃H₇)CH₂O(B)ₚ(A)ₙ(B)ₘ(A)_{q}H (I)

mit der Bedeutung
- A: Ethylenoxy
- B: Propylenoxy,
wobei Gruppen A und B in Form von Blöcken in der angegebenen Reihenfolge vorliegen,
- p: Zahl von 0 bis 5
- n: Zahl von 0,25 bis 10
- m: Zahl von 2 bis 10
- q: Zahl größer 0 bis 10 von 1 bis 5
wobei
- 70 bis 99 Gew.-%: Alkoxylate A1, in denen C₅H₁₁ die Bedeutung n-C₅H₁₁ hat, und
- 1 bis 30 Gew.-%: Alkoxylate A2, in denen C₅H₁₁ die Bedeutung C₂H₅CH(CH₃)CH₂ und/oder CH₃CH(CH₃)CH₂CH₂ hat,
im Gemisch vorliegen.

Es wurde erfindungsgemäß gefunden, dass die vorstehenden Alkoxylat-Gemische hervorragende Emulgatoreigenschaften zeigen und als nicht oder wenig schäumende Netzmittel für harte Oberflächen eingesetzt werden können. Die Alkoxylate zeigen geringe Kontaktwinkel bei der Benetzung harter Oberflächen und erlauben die Einstellung geringer Grenzflächenspannungen in flüssigen Systemen.

Damit sind die Alkoxylat-Gemische der allgemeinen Formel (I) besonders vorteilhaft einsetzbar, insbesondere als Emulgator, Schaumregulierer und als Netzmittel für harte Oberflächen in Tensidformulierungen zur Reinigung harter Oberflächen, in Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, in Emulsionspolymerisationen oder zur Herstellung von Additiven beziehungsweise Verwendung als Additive für mineralische Baustoffe (wie zum Beispiel Zementmörtel, Beton, Gips, Gipsestrich, Zementestrich etc.). Auf die einzelnen Anwendungsgebiete wird nachfolgend noch näher eingegangen.

In der allgemeinen Formel (I) bedeutet p eine Zahl von 0 bis 10, vorzugsweise 0 bis 5, insbesondere 0 bis 3. Sofern Blöcke (B)ₚ vorliegen, ist p vorzugsweise eine Zahl von 0,1 bis 10, besonders bevorzugt 0,5 bis 5, insbesondere 1 bis 3.

In der allgemeinen Formel (I) bedeutet n vorzugsweise eine Zahl im Bereich von 0,25 bis 10, insbesondere von 0,5 bis 7, m ist vorzugsweise eine Zahl im Bereich von 2 bis 10, insbesondere 3 bis 6. B ist vorzugsweise Propylenoxy und/oder Butylenoxy, speziell Propylenoxy an beiden Positionen.

q ist vorzugsweise eine Zahl im Bereich von 1 bis 5, besonders bevorzugt im Bereich von 2 bis 3.

Die Summe p + n + m + q ist mindestens 1, vorzugsweise 3 bis 25, besonders bevorzugt 5 bis 15, insbesondere 7 bis 13.

In den erfindungsgemäßen Alkoxylaten liegen 3 oder 4 Alkylenoxidblöcke vor. Gemäß einer Ausführungsform liegen an den Alkoholrest anschließend zunächst Ethylenoxy-Einheiten, daran anschließend Propylenoxid-Einheiten und daran anschließend Ethylenoxy-Einheiten vor. Gemäß einer weiteren Auführungsform liegen an den Alkoholrest anschließend zunächst Propylenoxy-Einheiten, so dann Ethylenoxy-Einheiten, so dann Propylenoxy-Einheiten und abschließend Ethylenoxy-Einheiten vor. Anstelle der Propylenoxy-Einheiten können auch die anderen angegebenen Alkylenoxy-Einheiten vorliegen.

p, n, m und q bezeichnen dabei einen mittleren Wert, der sich als Durchschnitt für die Alkoxylate ergibt. Daher können p, n, m, q auch von ganzzahligen Werten abweichen. Bei der Alkoxylierung von Alkanolen wird im Allgemeinen eine Verteilung des Alkoxylierungsgrades erhalten, die in gewissem Umfang durch Einsatz unterschiedlicher Alkoxylierungskatalysatoren eingestellt werden kann. Durch die Auswahl geeigneter Mengen der Gruppen A und B kann das Eigenschaftsspektrum der erfindungsgemäßen Alkoxylat-Gemische je nach praktischen Erfordernissen angepasst werden.

Die erfindungsgemäßen Alkoxylat-Gemische werden durch Alkoxylierung der zugrunde liegenden Alkohole C₅H₁₁CH(C₃H₇)CH₂OH erhalten. Die Ausgangsalkohole können aus den einzelnen Komponenten gemischt werden, so dass sich das erfindungsgemäße Verhältnis ergibt. Sie können durch Aldolkondensation von Valeraldehyd und nachfolgende Hydrierung hergestellt werden. Die Herstellung von Valeraldehyd und den entsprechenden Isomeren erfolgt durch Hydroformylierung von Buten, wie beispielsweise in US 4,287,370; Beilstein E IV 1, 32 68, Ullmanns Encyclopedia of Industrial Chemistry, 5. Auflage, Band A1, Seiten 323 und 328 f beschrieben. Die nachfolgende Aldolkondensation ist beispielsweise beschrieben in US 5,434,313 und Römpp, Chemie Lexikon, 9. Auflage, Stichwort "Aldol-Addition" Seite 91. Die Hydrierung des Aldolkondensationsproduktes folgt allgemeinen Hydrierbedingungen.

Des Weiteren kann 2-Propylheptanol durch Kondensation von 1-Pentanol (als Mischung der entsprechenden Methylbutanole-1) in Gegenwart von KOH bei erhöhten Temperaturen hergestellt werden, siehe z.B. Marcel Guerbet, C.R. Acad Sci Paris 128, 511, 1002 (1899). Des Weiteren ist auf Römpp, Chemie Lexikon, 9. Auflage, Georg Thieme Verlag Stuttgart, und die dort genannten Zitate sowie Tetrahedron, Vol. 23, Seiten 1723 bis 1733, hinzuweisen.

In der allgemeinen Formel (I) kann der Rest C₅H₁₁ die Bedeutung n-C₅H₁₁, C₂H₅CH(CH₃)CH₂ oder CH₃CH(CH₃)CH₂CH₂ haben. Es handelt sich bei den Alkoxylaten um Gemische, wobei

70 bis 99 Gew.-%, vorzugsweise 85 bis 96 Gew.-% Alkoxylate A1 vorliegen, in denen C₅H₁₁ die Bedeutung n-C₅H₁₁ hat, und
1 bis 30 Gew.-%, vorzugsweise 4 bis 15 Gew.-% Alkoxylate A2, in denen C₅H₁₁ die Bedeutung C₂H₅CH(CH₃)CH₂ und/oder CH₃CH(CH₃)CH₂CH₂ hat.

Der Rest C₃H₇ hat vorzugsweise die Bedeutung n-C₃H₇.

Vorzugsweise wird die Alkoxylierung durch starke Basen katalysiert, die zweckmäßigerweise in Form eines Alkalialkoholats, Alkalihydroxids oder Erdalkalihydroxids, in der Regel in einer Menge von 0,1 bis 1 Gew.-% bezogen auf die Menge des Alkanols R²-OH, zugesetzt werden, (vergl. G. Gee et al., J. Chem. Soc. (1961), S. 1345; B. Wojtech, Makromol. Chem. 66, (1966), S. 180).

Auch eine saure Katalyse der Additionsreaktion ist möglich. Neben Bronstedsäuren eignen sich auch Lewissäuren wie zum Beispiel AlCl₃ oder BF₃ Dietherat, BF₃, BF₃ x H₃PO₄, SbCl₄ × 2 H₂O, Hydrotalcit (Vgl. P.H. Plesch, The Chemistry of Cationic Polymerization, Pergamon Press, New York (1963). Geeignet als Katalysator sind auch Doppelmetallcyanid (DMC) Verbindungen.

Als DMC-Verbindung können prinzipiell alle dem Fachmann bekannten geeigneten Verbindungen verwendet werden.

Als Katalysator geeignete DMC-Verbindungen sind beispielsweise in der WO 99/16775 und der DE-A-101 17 273 beschrieben. Insbesondere sind für die Alkoxylierung Doppelmetallcyanid-Verbindung der allgemeinen Formel 1 als Katalysator geeignet:

M¹ₐ[M²(CN)_{b}(A)_{c}]_{d}·fM¹_{g}×ₙ· h(H₂O) eL·kP (I),

in der
- M¹ mindestens ein Metallion, ausgewählt aus der Gruppe bestehend aus Zn²⁺, Fe²⁺, Fe³⁺, Co³⁺, Ni²⁺, Mn²⁺, Co²⁺, Sn²⁺, Pb²⁺, Mo⁴⁺, Mo⁶⁺, Al³⁺, V⁴⁺, V⁵⁺, Sr²⁺, W⁴⁺, W⁶⁺, Cr²⁺, Cr³⁺, Cd²⁺, Hg²⁺, Pd²⁺, Pt²⁺, V²⁺, Mg²⁺, Ca²⁺, Ba²⁺, Cu²⁺, La³⁺, Ce³⁺, Ce⁴⁺, Eu³⁺, Ti³⁺, Ti⁴⁺, Ag⁺, Rh²⁺, Rh³⁺, Ru²⁺, Ru³⁺ ist,
- M² mindestens ein Metallion, ausgewählt aus der Gruppe bestehend aus Fe²⁺, Fe³⁺, Co²⁺, Co³⁺, Mn²⁺, Mn³⁺, V⁴⁺, V⁵⁺, Cr²⁺, Cr³⁺, Rh³⁺, Ru²⁺, Ir³⁺ist,
- A und X unabhängig voneinander ein Anion, ausgewählt aus der Gruppe, bestehend aus Halogenid, Hydroxid, Sulfat, Carbonat, Cyanid, Thiocyanat, Isocyanat, Cyanat, Carboxylat, Oxalat, Nitrat, Nitrosyl, Hydrogensulfat, Phosphat, Dihydrogenphosphat, Hydrogenphosphat oder Hydrogencarbonat sind,
- L ein mit Wasser mischbarer Ligand ist, ausgewählt aus der Gruppe, bestehend aus Alkoholen, Aldehyden, Ketonen, Ethern, Polyethern, Estern, Polyestern, Polycarbonat, Harnstoffen, Amiden, primären, sekundären und tertiären Aminen, Liganden mit Pyridin-Stickstoff, Nitrilen, Sulfiden, Phosphiden, Phosphiten, Phosphanen, Phosphonaten und Phosphaten,
- k eine gebrochene oder ganze Zahl größer oder gleich Null ist, und
- P ein organischer Zusatzstoff ist,
- a, b, c, d, g und n so ausgewählt sind, dass die Elektroneutralität der Verbindung (1) gewährleistet ist, wobei c = 0 sein kann,
- e die Anzahl der Ligandenmoleküle eine gebrochene oder ganze Zahl größer 0 oder 0 ist,
- f und h unabhängig voneinander eine gebrochene oder ganze Zahl größer 0 oder 0 sind.

Als organische Zusatzstoffe P sind zu nennen: Polyether, Polyester, Polycarbonate, Polyalkylenglykolsorbitanester, Polyakylenglykolglycidylether, Polyacrylamid, Poly(acrylamid-co-acrylsäure), Polyacrylsäure, Poly(acrylamid-co-maleinsäure), Polyacrylnitril, Polyalkylacrylate, Polyalkylmethacrylate, Polyvinylmethylether, Polyvinylethylether, Polyvinylacetat, Polyvinylalkohol, Poly-N-vinylpyrrolidon, Poly(N-vinylpyrrolidon-co-acrylsäure), Polyvinylmethylketon, Poly(4-vinylphenol), Poly(acrylsäure-co-styrol),

Oxazolinpolymere, Polyalkylenimine, Maleinsäure- und Maleinsäureanhydridcopolymere, Hydroxyethylcellulose, Polyacetate, ionische oberflächen- und grenzflächenaktive Verbindungen, Gallensäure oder deren Salze, Ester oder Amide, Carbonsäureester mehrwertiger Alkohole und Glycoside.

Diese Katalysatoren können kristallin oder amorph sein. Für den Fall, dass k gleich null ist, sind kristalline Doppelmetallcyanid-Verbindungen bevorzugt. Im Fall, dass k größer null ist, sind sowohl kristalline, teilkristalline, als auch substantiell amorphe Katalysatoren bevorzugt.

Von den modifizierten Katalysatoren gibt es verschiedene bevorzugte Ausführungsformen. Eine bevorzugte Ausführungsform sind Katalysatoren der Formel (1), bei denen k größer null ist. Der bevorzugte Katalysator enthält dann mindestens eine Doppelmetallcyanid-Verbindung, mindestens einen organischen Liganden und mindestens einen organischen Zusatzstoff P.

Bei einer anderen bevorzugten Ausführungsform ist k gleich null, optional ist e auch gleich null und X ist ausschließlich ein Carboxylat, bevorzugt Formiat, Acetat und Propionat. Derartige Katalysatoren sind in der WO 99/16775 beschrieben. Bei dieser Ausführungsform sind kristalline Doppelmetallcyanid-Katalysatoren bevorzugt. Ferner bevorzugt sind Doppelmetallcyanid-Katalysatoren, wie in der WO 00/74845 beschrieben, die kristallin und plättchenförmig sind.

Die Herstellung der modifizierten Katalysatoren erfolgt durch Vereinigung einer Metallsalz-Lösung mit einer Cyanometallat-Lösung, die optional sowohl einen organischen Liganden L als auch einen organischen Zusatzstoff P enthalten können. Anschließend werden der organische Ligand und optional der organische Zusatzstoff zugegeben. Bei einer bevorzugten Ausführungsform der Katalysatorherstellung wird zunächst eine inaktive Doppelmetallcyanid-Phase hergestellt und diese anschließend durch Umkristallisation in eine aktive Doppelmetallcyanidphase überführt, wie in der PCT/EP01/01893 beschrieben.

Bei einer anderen bevorzugten Ausführungsform der Katalysatoren sind f, e und k ungleich Null. Dabei handelt es sich um Doppelmetallcyanid-Katalysatoren, die einen mit Wasser mischbaren organischen Ligand (im Allgemeinen in Mengen von 0,5 bis 30 Gew.%) und einen organischen Zusatzstoff (im allgemeinen in Mengen von 5 bis 80 Gew.%) enthalten wie in der WO 98/06312 beschrieben. Die Katalysatoren können entweder unter starkem Rühren (24000U/Min mit Turrax) oder unter Rühren hergestellt werden wie in der US 5,158,922 beschrieben.

Insbesondere als Katalysator geeignet sind für die Alkoxylierung Doppelmetallcyanid-Verbindungen, die Zink, Kobalt oder Eisen oder zwei davon enthalten. Besonders geeignet ist beispielsweise Berliner Blau.

Bevorzugt werden kristalline DMC-Verbindungen eingesetzt. In einer bevorzugten Ausführungsform wird eine kristalline DMC-Verbindung vom Zn-Co-Typ als Katalysator verwendet, der als weitere Metallsalzkomponente Zinkacetat enthält. Derartige Verbindungen kristallisieren in monokliner Struktur und weisen einen plättchenförmigen Habitus auf. Derartige Verbindungen werden beispielsweise in der WO 00/74845 oder der PCT/EP01/01893 beschrieben.

Als Katalysator geeignete DMC-Verbindungen können prinzipiell auf alle dem Fachmann bekannten Arten hergestellt werden. Beispielsweise können die DMC-Verbindungen durch direkte Fällung, "incipient wetness"-Methode, durch Herstellung einer Precursor-Phase und anschließende Umkristallisation hergestellt werden.

Die DMC-Verbindungen können als Pulver, Paste oder Suspension eingesetzt werden oder zu einem Formkörper verformt werden, in Formkörpern, Schäume oder ähnliches eingebracht werden oder auf Formkörper, Schäume oder ähnliches aufgebracht werden.

Die zur Alkoxylierung eingesetzte Katalysator-Konzentration, bezogen auf das Endmengengerüst ist typischerweise kleiner als 2000 ppm (d.h. mg Katalysator pro kg Produkt), bevorzugt kleiner als 1000 ppm, insbesondere kleiner als 500 ppm, besonders bevorzugt kleiner als 100 ppm, beispielsweise kleiner als 50 ppm oder 35 ppm, insbesondere bevorzugt kleiner als 25 ppm.

Die Additionsreaktion wird bei Temperaturen von 90 bis 240°C, vorzugsweise von 120 bis 180°C, im geschlossenen Gefäß ausgeführt. Das Alkylenoxid oder die Mischung verschiedener Alkylenoxide wird der Mischung aus erfindungsgemäßen Alkanolgemisch und Alkali unter dem bei der gewählten Reaktionstemperatur herrschenden Dampfdruck des Alkylenoxidgemisches zugeführt. Gewünschtenfalls kann das Alkylenoxid mit bis zu etwa 30 bis 60 % mit einem Inertgas verdünnt werden. Dadurch wird eine zusätzliche Sicherheit gegen explosionsartige Polyaddition des Alkylenoxids gegeben.

Wird ein Alkylenoxidgemisch eingesetzt, so werden Polyetherketten gebildet, in denen die verschiedenen Alkylenoxidbausteine praktisch statistisch verteilt sind. Variationen in der Verteilung der Bausteine längs der Polyetherkette ergeben sich aufgrund unterschiedlicher Reaktionsgeschwindigkeiten der Komponenten und können auch willkürlich durch kontinuierliche Zufuhr einer Alkylenoxidmischung programmgesteuerter Zusammensetzung erreicht werden. Werden die verschiedenen Alkylenoxide nacheinander zur Reaktion gebracht, so erhält man Polyetherketten mit blockartiger Verteilung der Alkylenoxid-Bausteine

Die Länge der Polyetherketten schwankt innerhalb des Reaktionsprodukts statistisch um einen Mittelwert, der im Wesentlichen sich aus der Zusatzmenge ergebenden stöchiometrischen Wert.

Bevorzugte Alkoxylat-Gemische der allgemeinen Formel (1) können erfindungsgemäß erhalten werden durch Umsetzung von Alkoholen der allgemeinen Formel C₅H₁₁CH(C₃H₇)CH₂OH mit Propylenoxid/Ethylenoxid in der vorstehend angegebenen Reihenfolge unter Alkoxylierungsbedingungen. Geeignete Alkoxylierungsbedingungen sind vorstehend und in Nikolaus Schönfeldt, Grenzflächenaktive Äthylenoxid-Addukte, Wissenschaftliche Verlagsgesellschaft mbH Stuttgart 1984 beschrieben. In der Regel wird die Alkoxylierung in Gegenwart basischer Katalysatoren wie KOH in Substanz durchgeführt. Die Alkoxylierung kann jedoch auch unter Mitverwendung eines Lösungsmittels durchgeführt werden. Dabei wird eine Polymerisation des Alkylenoxids in Gang gesetzt, bei der es zwangsläufig zu einer statistischen Verteilung von Homologen kommt, deren Mittelwert vorliegend mit p, n, m und q angegeben wird.

Bei einer erfindungsgemäß bevorzugt zunächst durchgeführten Propoxylierung und erst nachfolgenden Ethoxylierung kann der Gehalt an Restalkohol in den Alkoxylaten vermindert werden, da Propylenoxid gleichmäßiger an die Alkoholkomponente addiert wird. Im Unterschied dazu reagiert Ethylenoxid vorzugsweise mit Ethoxylaten, so dass bei einer anfänglichen Verwendung von Ethylenoxid zur Umsetzung mit den Alkanolen eine breitere Homologenverteilung resultieren kann. Die erfindungsgemäß eingesetzten Alkohol-Gemische haben in der Regel einen Eigengeruch, der durch die vollständige Alkoxylierung weitestgehend unterdrückt werden kann. Nach üblichen Verfahren erhaltene Alkoxylate weisen oftmals einen Eigengeruch auf, der für viele Anwendungen störend ist.

Die erfindungsgemäßen Alkoxylat-Gemische erfordern zum Absenken des Restalkohöl-Gehaltes nur einen, vorzugsweise direkt an den Alkohol gebundenen, Propylenoxid(PO)-Block von sehr kurzer Länge. Dies ist insbesondere deshalb sehr vorteilhaft, weil die biologische Abbaubarkeit des Produktes bei Verlängerung des PO-Blocks sinkt. Derartige Alkoxylat-Gemische ermöglichen somit maximale Freiheitsgrade bei der Wahl der Länge des PO-Blockes, wobei die Länge nach unten durch den steigenden Restalkoholgehalt und nach oben durch die Verschlechterung der biologischen Abbaubarkeit begrenzt ist.

Es ist erfindungsgemäß nicht notwendig, dass ein großer Restgehalt an Alkohol in den erfindungsgemäßen Alkoxylat-Gemischen vorliegt. Gemäß einer Ausführungsform der Erfindung weisen die Alkoxylat-Gemische einen verminderten oder keinen Anteil an Alkoholen auf.

Die erfindungsgemäßen Alkoxylat-Gemische zeigen eine verbesserte Netzung auf harten Oberflächen.

Das vorteilhafte Netzungsverhalten der erfindungsgemäßen Gemische kann beispielsweise durch Messungen des Kontaktwinkels auf Glas, Polyethylenoxid oder Stahl ermittelt werden. Aus dem verbesserten Netzungsverhalten folgt eine bessere Performance bei insbesondere schnellen Reinigungsprozessen. Dies ist insofern überraschend, da durch die Kettenverlängerung des Ausgangsalkohols üblicherweise die dynamischen und netzenden Eigenschaften vermindert werden. Mit den erfindungsgemäßen Alkoxylat-Gemischen kann damit die Benetzungsgeschwindigkeit von wässrigen Formulierungen erhöht werden. Die erfindungsgemäßen Alkoxylat-Gemische können damit auch als Solubilisatoren eingesetzt werden, die insbesondere das Netzvermögen von Netzhilfsmitteln auch in verdünnten Systemen nicht negativ, sondern positiv beeinflussen. Sie können zur Erhöhung der Löslichkeit von Netzhilfsmitteln in wässrigen Formulierungen eingesetzt werden, die nicht-ionische Tenside enthalten. Sie dienen insbesondere zur Erhöhung der Benetzungsgeschwindigkeit in wässrigen Netzmitteln.

Ferner dienen die erfindungsgemäßen Alkoxylat-Gemische zur Verminderung der Grenzflächenspannung, beispielsweise in wässrigen Tensidformulierungen. Die verminderte Grenzflächenspannung kann beispielsweise durch die Pendant-Drop-Methode bestimmt werden. Hieraus ergibt sich auch eine bessere Wirkung der erfindungsgemäßen Alkoxylat-Gemische als Emulgator oder Co-Emulgator. Die erfindungsgemäßen Alkoxylat-Gemische können auch zur Verminderung der Grenzflächenspannung bei kurzen Zeiten von üblicherweise unter einer Sekunde bzw. zur Beschleunigung der Einstellung der Grenzflächenspannung in wässrigen Tensidformulierungen eingesetzt werden.

Die vorliegende Erfindung betrifft auch Reinigungs-, Netz-, Beschichtungs-, Klebe-, Lederentfettungs-, Feuchthalte- oder Textilbehandlungsmittel, Ausgangsstoffe zur Herstellung von Additiven für mineralische Baustoffe beziehungsweise Additive für mineralische Baustoffe oder kosmetische, pharmazeutische oder Pflanzenschutzformulierungen, die mindestens ein wie vorstehend definiertes Alkoxylat-Gemische der allgemeinen Formel (I) enthalten. Die Mittel enthalten dabei vorzugsweise 0,1 bis 20 Gew.-% der Alkoxylat-Gemische. Nachstehend werden bevorzugte Einsatzgebiete der erfindungsgemäßen Alkoxylat-Gemische näher beschrieben. Die erfindungsgemäßen Alkoxylat-Gemische werden vorzugsweise in den folgenden Bereichen eingesetzt:
- Tensidformulierungen zur Reinigung harter Oberflächen: Geeignete Tensidformulierungen, die mit den erfindungsgemäßen Alkoxylaten additiviert werden können, sind beispielsweise in Formulating Detergents and Personal Care Products von Louis Ho Tan Tai, AOCS Press, 2000 beschrieben.
   Sie enthalten beispielsweise als weitere Komponenten Seife, anionische Tenside wie LAS oder Paraffinsulfonate oder FAS oder FAES, Säure wie Phosphorsäure, Amidosulfonsäure, Zitronensäure, Milchsäure, Essigsäure, andere organische und anorganische Säuren, Lösungsmittel wie Ethylenglykol, Isopropanol, Komplexbildner wie EDTA, NTA, MGDA, Phosphonate, Polymere wie Polyacrylate, Copolymere Maleinsäure-Acrylsäure, Alkalispender wie Hydroxide, Silicate, Carbonate, Parfümöle, Oxidationsmittel wie Perborate, Persäuren oder Trichloroisocyanursäure, Na- oder K-Dichlorisocyanurate, Enzyme; siehe auch Milton J. Rosen, Manilal Dahanayake, Industrial Utilization of Surfactants, A-OCS Press, 2000 und Nikolaus Schönfeldt, Grenzflächenaktive Ethylenoxidaddukte. Hier sind auch Formulierungen für die anderen genannten Anwendungen im Prinzip abgehandelt Es kann sich um Haushaltsreiniger wie Allzweckreiniger, Geschirrspülmittel für manuelles wie automatisches Geschirrspülen, Metallentfettung, Industrielle Applikationen wie Reinigungsmittel für die Nahrungsmittelindustrie Flaschenwäsche, etc. handeln. Es kann sich auch um Druckwalzen- und Druckplattenreinigungsmittel in der Druckindustrie handeln. Geeignete weitere Inhaltsstoffe sind dem Fachmann bekannt.
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Formulierungen für die Textilindustrie wie Egalisiermittel oder Formulierungen zur Garnreinigung.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Solche Formulierungen enthalten üblicherweise Inhaltsstoffe wie Tenside, Gerüst-, Duft- und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 und in Schönfeldt, s. o., beispielhaft beschrieben.

Allgemein können die erfindungsgemäßen Alkoxylat-Gemische in allen Bereichen eingesetzt werden, in denen die Wirkung von grenzflächenaktiven Stoffen notwendig ist.

Die erfindungsgemäßen Strukturen weisen eine gegenüber bekannten Strukturen, geringe Aquatoxizität und leichte biologische Abbaubarkeit auf, so dass sie für eine Vielzahl von Anwendungsgebieten vorteilhaft geeignet sind.

Im folgenden soll die vorliegende Erfindung anhand von Beispielen näher erläutert werden.

### BEISPIELE

Als 2-Propyl-Heptanol-1 wurde ein Isomerengemisch aus 87% 2-Propylheptanol-1, 11 % 2-Propyl-4-methylhexanol-1 und < 1 % 2-Propyl-5-methylhexanol-1 eingesetzt.

### Beispiel 1: Alkoxylierung des 2-Propylheptanol-Isomerengemisches mit EO, PO und EO mittels KOH-Katalyse

### 1.1 2-Propylheptanol + 5,2 EO + 4.7 PO + 2,3 EO

158,3 g (1,0 Mol) 2-Propylheptanol-Isomerengemisch und 3,8 g 40 Gew.-%-ige Kalilauge wurden vermischt und in einem Autoklaven bei 80 °C und < 10 mbar über 30 Minuten entwässert. Der Autoklav wurde mit Stickstoff inertisiert und dann auf 145 - 155 °C erwärmt. Zunächst wurden zum Aufbau des ersten EO-Blocks 228,8 g (5,2 Mol) Ethylenoxid zudosiert und etwa 1 Stunde bis zur Druckkonstanz nachreagieren lassen. Anschließend wurde die Innentemperatur des Autoklaven auf 125 - 135 °C abgesenkt, nachfolgen zum Aufbau des PO-Blocks 272,6 g (4,7 Mol) Propylenoxid zudosiert und etwa 5 Stunden bis zur Druckkonstanz nachreagieren lassen. Schließlich wurde die Innentemperatur wieder auf 145 - 155°C erhöht, zum Aufbau des zweiten EO-Blocks 101,2 g (2,3 Mol) Ethylenoxid zudosiert und etwa 1 Stunde bis zur Druckkonstanz nachreagieren lassen. Zum Entgasen wurde evakuiert, nachfolgend das Reaktionsprodukt durch Zugabe von Essigsäure bei 80 °C auf einen pH-Wert zwischen 6 und 7 eingestellt und schließlich der Reaktor entleert.

Das Produkt wies folgende Eigenschaften auf:
OHZ soll: 69,6 OHZ ist: 71,0
Netzung auf textilen Oberflächen (EN 1772): 17 sec (23 °C, 1 g/l in 2 g Soda/l)
- Schaumvermögen (EN 122728):: ca. 15 ml (40 °C; 2 g/l; 1,8 mmol Ca2+- Ionen, nach 30 sec)
- Oberflächenspannung (DIN 53914):: ca. 28,2 mN/m (1 g/l; 23 °C)

### 1.2 2-Propylheptanol + 0,7 EO + 4,7 PO + 2,3 EO

Die Herstellung erfolgte analog dem Beispiel 1.1. Es wurden 158,3 g (1,0 Mol) 2-Propylheptanol-Isomerengemisch, für den ersten EO-Block 30,8 g (0,7 Mol) Ethylenoxid, für den PO-Block 272,6 g (4,7 Mol) Propylenoxid, für den zweiten EO-Block 101,2 g (2,3 Mol) Ethylenoxid und 2,8 g 40 Gew.-%-ige Kalilauge verwendet.

Das Produkt wies folgende Eigenschaften auf:
OHZ soll: 99,5 OHZ ist: 97,0
- Netzung auf textilen Oberflächen (EN 1772):: 45 sec (23°C, 1g/l in 2 g Soda/l)
- Schäumvermögen (EN 122728):: ca. 10 ml (40°C; 2 g/l; 1,8 mmol Ca2+- Ionen, nach 30 sec)
- Oberflächenspannung (DIN 53914):: ca. 27,8 mN/m (1 g/l; 23°C)

### 1.3 2-Propylheptanol + 3,2 EO + 2,7 PO + 2,3 EO

Die Herstellung erfolgte analog dem Beispiel 1.1. Es wurden 158,3 g (1,0 Mol) 2-Propylheptanol-Isomerengemisch, für den ersten EO-Block 140,8 g (3,2 Mol) Ethylenoxid, für den PO-Block 156,6 g (2,7 Mol) Propylenoxid, für den zweiten EO-Block 101,2 g (2,3 Mol) Ethylenoxid und 2,78 g 40 Gew.-%-ige Kalilauge verwendet.

Das Produkt wies folgende Eigenschaften auf:
OHZ soll: 100,6 OHZ ist: 101,0
- Netzung auf textilen Oberflächen (EN 1772):: 15 sec (23 °C, 1 g/l in 2 g Soda/l)
- Schaumvermögen (EN 122728):: ca. 15 ml (40 °C; 2 g/l; 1,8 mmol Ca2+- Ionen
- Oberflächenspannung (DIN 53914):: ca. 27,0 mN/m (1 g/l; 23 °C)

### 1.4 2-Propylheptanol + 2,7 EO + 3,0 PO + 2,3 EO

Die Herstellung erfolgte analog dem Beispiel 1.1. Es wurden 158,3 g (1,0 Mol) 2-Propylheptanol-Isomerengemisch, für den ersten EO-Block 118,8 g (2,7 Mol) Ethylenoxid, für den PO-Block 174,0 g (3,0 Mol) Propylenoxid, für den zweiten EO-Block 101,2 g (2,3 Mol) Ethylenoxid und 2,76 g 40 Gew.-%-ige Kalilauge verwendet.

Das Produkt wies folgende Eigenschaften auf:
OHZ soll: 101,5 OHZ ist: 102,4
- Netzung auf textilen Oberflächen (EN 1772):: 23 sec (23 °C, 1 g/l in 2 g Soda/l)
- Schäumvermögen (EN 122728):: ca. 10 ml (40 °C; 2 g/l; 1,8 mmol Ca2+- Ionen, nach 30 sec)
- Oberflächenspannung (DIN 53914):: ca. 25,5 mN/m (1 g/l; 23 °C)

### 1.5 2-Propylheptanol + 2,7 EO + 6,0 PO + 2,3 EO

Die Herstellung erfolgte analog dem Beispiel 1.1. Es wurden 158,3 g (1,0 Mol) 2-Propylheptanol-Isomerengemisch, für den ersten EO-Block 118,8 g (2,7 Mol) Ethylenoxid, für den PO-Block 348,0 g (6,0 Mol) Propylenoxid, für den zweiten EO-Block 101.2 g (2,3 Mol) Ethylenoxid und 3,63 g 40 Gew.-%ige Kalilauge verwendet.

Das Produkt wies folgende Eigenschaften auf:
OHZ soll: 77,1 OHZ ist: 81,5
- Netzung auf textilen Oberflächen (EN 1772):: 45 sec (23 °C, 1 g/l in 2 g Soda/l).
- Schäumvermögen (EN 122728):: < 10 ml (40 °C; 2 g/l; 1,8 mmol Ca2+- Ionen, nach 30 sec)
- Oberflächenspannung (DIN 53914):: ca. 28,3 mN/m (1 g/l; 23 °C)

### Beispiel 2: Alkoxylierung des 2-Propylheptanol-Isomerengemisches mit PO, EO, PO und EO mittels KOH-Katalyse

### 2.1 2-Propylheptanol + 1,5 PO + 2,7 EO + 4,7 PO + 2,3 EO

158,3 g (1,0 Mol) 2-Propylheptanol-Isomerengemisch und 3,7 g 40 Gew.-%-ige Kalilauge wurden vermischt und in einem Autoklaven bei 80 °C und < 10 mbar über 30 Minuten entwässert. Der Autoklav wurde mit Stickstoff inertisiert und dann auf 125 - 130 °C erwärmt. Zunächst wurden zum Aufbau des ersten PO-Blocks 87,0 g (1,5 Mol) Propylenoxid zudosiert und etwa 1,75 Stunden nachreagieren lassen. Die Innentemperatur des Autoklaven wurde dann auf 145 - 155°C erhöht und zum Aufbau des ersten EO-Blocks 118,8g (2,7 Mol) Ethylenoxid zudosiert und 45 Minuten bis zur Druckkonstanz nachreagieren lassen. Anschließend wurde die Innentemperatur des Autoklaven auf 125 - 135 °C abgesenkt, nachfolgend zum Aufbau des zweiten PO-Blocks 272,6 g (4,7 Mol) Propylenoxid zudosiert und etwa 1,75 Stunden bis zur Druckkonstanz nachreagieren lassen. Schließlich wurde die Innentemperatur wieder auf 145 - 155 °C erhöht, zum Aufbau des zweiten EO-Blocks 101,2 g (2,3 Mol) Ethylenoxid zudosiert und etwa 1 Stunde bis zur Druckkonstanz nachreagieren lassen. Zum Entgasen wurde evakuiert, nachfolgend das Reaktionsprodukt durch Zugabe von Essigsäure bei 80 °C auf einen pH-Wert zwischen 6 und 7 eingestellt und schließlich der Reaktor entleert.

Das Produkt wies folgende Eigenschaften auf:
OHZ soll: 75,9 OHZ ist: 76,1
- Netzung auf textilen Oberflächen (EN 1772):: 39 sec (23 °C, 1 g/l in 2 g Soda/l)
- Schäumvermögen (EN 122728):: ca. 10 ml (40 °C; 2 g/l; 1,8 mmol Ca2+- Ionen, nach 30 sec)
- Oberflächenspannung (DIN 53914):: ca. 28,1 mN/m (1 g/l; 23°C)

### 2.2 2-Propylheptanol + 1,5 PO + 1,7 EO + 4,7 PO + 2,3 EO

158,3 g (1,0 Mol) 2-Propylheptanol-Isomerengemisch und 3,47 g 40 Gew.-%-ige Kalilauge wurden vermischt und in einem Autoklaven bei 80 °C und < 10 mbar über 30 Minuten entwässert. Der Autoklav wurde mit Stickstoff inertisiert und dann auf 125 - 130 °C erwärmt. Zunächst wurden zum Aufbau des ersten PO-Blocks 87,0 g (1,5 Mol) Propylenoxid zudosiert und etwa 2 Stunden nachreagieren lassen. Die Innentemperatur des Autoklaven wurde dann auf 145 - 155 °C erhöht und zum Aufbau des ersten EO-Blocks 74,8 g (1,7 Mol) Ethylenoxid zudosiert und 45 Minuten bis zur Druckkonstanz nachreagieren lassen. Anschließend wurde die Innentemperatur des Autoklaven auf 125 - 135 °C abgesenkt, nachfolgend zum Aufbau des zweiten PO-Blocks 272,6 g (4,7 Mol) Propylenoxid zudosiert und etwa 2 Stunden bis zur Druckkonstänz nachreagieren lassen. Schließlich wurde die Innentemperatur wieder auf 145 - 155 °C erhöht, zum Aufbau des zweiten EO-Blocks 101,2 g (2,3 Mol) Ethylenoxid zudosiert und etwa 1 Stunde bis zur Druckkonstanz nachreagieren lassen. Zum Entgasen wurde evakuiert, nachfolgend das Reaktionsprodukt durch Zugabe von Essigsäure bei 80 °C auf einen pH-Wert zwischen 6 und 7 eingestellt und schließlich der Reaktor entleert.

Das Produkt wies folgende Eigenschaften auf:
OHZ soll: 80,7 OHZ ist: 79,4
- Netzung auf textilen Oberflächen (EN1772):: 61 sec (23 °C, 1 g/l in 2 g Soda/l)
- Schäumvermögen (EN 122728):: ca. 10 ml (40 °C; 2 g/l; 1,8 mmol Ca2+-lonen, nach 30 sec)
- Oberflächenspannung (DIN 53914):: ca. 28,2 mN/m (1 g/l; 23 °C)

Weitere Alkoxylate sind nachfolgend tabellarisch zusammengefasst:

| **EO [moleq.]** | **PO [moleq.]** | **EO [moleq.]** | **M (theor.)** | **OHZ soll** | **OHZ ist** |
|---|---|---|---|---|---|
| 0,70 | 0,70 | 0,30 | 243,00 | 230,9 | 232,9 |
| 0,70 | 0,70 | 2,30 | 331,10 | 169,4 | 166,8 |
| 0,70 | 2,70 | 1,30 | 403,21 | 139,1 | 133,8 |
| 1,70 | 4,70 | 2,30 | 607,47 | 92,3 | 94,8 |
| 2,70 | 4,70 | 2,30 | 651,52 | 86,1 | 86,0 |
| 3,20 | 0,70 | 1,30 | 397,17 | 141,2 | 136,9 |
| 3,20 | 2,70 | 0,30 | 469,28 | 119,5 | 116,1 |
| 3,20 | 2,70 | 1,30 | 513,33 | 109,3 | 109,4 |
| 3,20 | 4,70 | 1,30 | 629,49 | 89,1 | 90,3 |
| 3,70 | 4,70 | 2,30 | 695,57 | 80,6 | 80,4 |
| 4,70 | 4,70 | 2,30 | 739,62 | 75,8 | 74,8 |
| 5,70 | 0,70 | 0,30 | 463,25 | 121,1 | 120,4 |
| 5,70 | 0,70 | 2,30 | 551,35 | 101,7 | 104,9 |
| 5,70 | 2,70 | 1,30 | 623,46 | 90,0 | 88,6 |
| 5,70 | 4,70 | 0,30 | 695,57 | 80,6 | 75,9 |
| 5,70 | 4,70 | 2,30 | 783,67 | 71,6 | 72,9 |

## Patentansprüche

1. Alkoxylat-Gemische, enthaltend Alkoxylate der allgemeinen Formel (I)
C₅H₁₁CH(C₃H₇)CH₂O(B)ₚ(A)ₙ(B)ₘ(A)_{q}H (I)
mit der Bedeutung
A Ethylenoxy
B Propylenoxy,
wobei Gruppen A und B in Form von Blöcken in der angegebenen Reihenfolge vorliegen,
p Zahl von 0 bis 5
n Zahl von 0,25 bis 10,
m Zahl von 2 bis 10
q Zahl von 1 bis 5
wobei
70 bis 99 Gew.-% Alkoxylate A1, in denen C₅H₁₁ die Bedeutung n-C₅H₁₁ hat, und
1 bis 30 Gew.-% Alkoxylate A2, in denen C₅H₁₁ die Bedeutung C₂H₅CH(CH₃)CH₂ und/oder CH₃CH(CH₃)CH₂CH₂ hat,
im Gemisch vorliegen.

2. Alkoxylat-Gemische nach Anspruch 1, **dadurch gekennzeichnet, dass** C₃H₇ die Bedeutung n-C₃H₇ hat.

3. Alkoxylat-Gemische nach Anspruch 1 oder 2, **dadurch gekennzeichnet, dass** in der allgemeinen Formel (I) p eine Zahl von 0,5 bis 5 ist.

4. Alkoxylat-Gemische nach einem der Ansprüche 1 bis 3, **dadurch gekennzeichnet, dass** 85 bis 96 Gew.-% Alkoxylate A1 und 4 bis 15 Gew.-% Alkoxylate A2 vorliegen.

5. Verfahren zur Herstellung von Alkoxylat-Gemischen nach einem der Ansprüche 1 bis 4 durch Umsetzung des Alkoholgemisches mit C₂₋₅-Alkylenoxiden unter Alkoxylierungsbedingungen.

6. Verfahren zur Herstellung von Alkoxylat-Gemischen nach Anspruch 5, **dadurch gekennzeichnet, dass** die Alkoxylierung in Gegenwart einer Doppelmetallcyanid-Verbindung als Katalysator erfolgt.

7. Verwendung von Alkoxylat-Gemischen gemäß einem der Ansprüche 1 bis 4 als Emulgator, Schaumregulierer und als Netzmittel für harte Oberflächen.

8. Verwendung nach Anspruch 7 in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, in Emulsionspolymerisationen und als Additive oder Ausgangsstoff zur Herstellung von Additiven für mineralische Baustoffe.

9. Wasch-, Reinigungs-, Netz-, Beschichtungs-, Klebe-, Lederfettungs-, Feuchthalte- oder Textilbehandlungsmittel, Additive für mineralische Baustoffe oder kosmetische, pharmazeutische oder Pflanzenschutzformulierungen, enthaltend Alkoxylat-Gemische gemäß einem der Ansprüche 1 bis 4.

## Claims

1. An alkoxylate mixture comprising alkoxylates of the formula (I)
C₅H₁₁CH(C₃H₇)CH₂O(B)ₚ(A)ₙ(B)ₘ(A)_{q}H (I)
where
A is ethyleneoxy,
B is propyleneoxy
groups A and B being present in the form of blocks in the stated sequence,
p is a number from 0 to 5,
n is a number from 0.25 to 10,
m is a number from 2 to 10,
q is a number from 1 to 5,
from 70 to 99% by weight of alkoxylates A1, in which C₅H₁₁ is n-C₅H₁₁, and
from 1 to 30% by weight of alkoxylates A2, in which C₅H₁₁ is C₂H₅CH(CH₃)CH₂ and/or CH₃CH(CH₃)CH₂CH₂,
being present in the mixture.

2. The alkoxylate mixture according to claim 1, wherein C₃H₇ is n-C₃H₇.

3. The alkoxylate mixture according to claim 1 or 2, wherein, in the formula (I), p is a number from 0.5 to 5.

4. The alkoxylate mixture according to any of claims 1 to 3, wherein from 85 to 96% by weight of alkoxylates A1 and from 4 to 15% by weight of alkoxylates A2 are present.

5. A process for the preparation of alkoxylate mixtures according to any of claims 1 to 4 by reacting the alcohol mixture with C₂₋₅-alkylene oxides under alkoxylation conditions.

6. The process for the preparation of alkoxylate mixtures according to claim 5, wherein the alkoxylation is carried out in the presence of a double metal cyanide compound as a catalyst.

7. The use of alkoxylate mixtures according to any of claims 1 to 4 as an emulsifier, foam regulator and wetting agent for hard surfaces.

8. The use according to claim 7 in detergents, surfactant formulations for cleaning hard surfaces, humectants, cosmetic, pharmaceutical and crop protection formulations, finishes, coating materials, adhesives, leather fatliquoring agents, formulations for the textile industry, fiber processing, metal processing, food industry, water treatment, paper industry, fermentation or mineral processing, in emulsion polymerizations and as additives or starting materials for the preparation of additives for mineral building materials.

9. A detergent, cleaning agent, wetting agent, coating material, adhesive, leather fatliquoring agent, humectant or textile treatment composition, additive for mineral building materials or cosmetic, pharmaceutical or crop protection formulation comprising alkoxylate mixtures according to any of claims 1 to 4.

## Revendications

1. Mélanges d'alcoxylates, contenant des alcoxylates de formule générale (I) :
C₅H₁₁CH(C₃H₇)CH₂O(B)ₚ(A)ₙ(B)ₘ(A)_{q}H (I)
avec la signification suivante :
A représente un groupement éthylénoxy,
B représente un groupement propylénoxy,
les groupements A et B se présentant sous la forme de séquences selon l'ordre indiqué,
p est un nombre de 0 à 5,
n est un nombre de 0,25 à 10,
m est un nombre de 2 à 10,
q est un nombre de 1 à 5,
dans lesquels :
70 à 99 % en poids des alcoxylates A1, dans lesquels C₅H₁₁ signifie n-C₅H₁₁, et
1 à 30 % en poids des alcoxylates A2, dans lesquels C₅H₁₁ signifie C₂H₅CH (CH₃) CH₂ et/ou CH₃CH (CH₃) CH₂CH₂, sont présents en mélange.

2. Mélanges d'alcoxylates selon la revendication 1, **caractérisés en ce que** C₃H₇ signifie n-C₃H₇.

3. Mélanges d'alcoxylates selon la revendication 1 ou 2, **caractérisés en ce que** p représente, dans la formule générale (I), un nombre de 0,5 à 5.

4. Mélanges d'alcoxylates selon l'une quelconque des revendications 1 à 3, **caractérisés en ce qu'**ils contiennent 85 à 96 % en poids d'alcoxylates A1 et 4 à 15 % en poids d'alcoxylates A2.

5. Procédé de fabrication de mélanges d'alcoxylates selon l'une quelconque des revendications 1 à 4, en faisant réagir le mélange d'alcools avec des oxydes d'alkylène en C₂-C₅ dans des conditions d'alcoxylation.

6. Procédé de fabrication de mélanges d'alcoxylates selon la revendication 5, **caractérisé en ce que** l'on effectue l'alcoxylation en présence d'un composé de cyanure doublement métallique comme catalyseur.

7. Utilisation de mélanges d'alcoxylates selon l'une quelconque des revendications 1 à 4, comme agents émulsionnants, régulateurs de mousse et comme agents mouillants pour des surfaces dures.

8. Utilisation selon la revendication 7 dans des agents de lavage, des formulations tensioactives pour le nettoyage de surfaces dures, des agents humidifiants, des formulations cosmétiques, pharmaceutiques et phytosanitaires, des vernis, des agents d'enduction, des colles, des agents nourrissants pour le cuir, des formulations pour l'industrie textile, dans la transformation des fibres, dans la transformation des métaux, dans l'industrie alimentaire, dans le traitement de l'eau, dans l'industrie du papier, dans la fermentation, dans la transformation des minéraux, dans des polymérisations en émulsion et comme additifs ou substance de départ pour la fabrication d'additifs pour des matériaux de construction minéraux.

9. Agents de lavage, de nettoyage, de mouillage, d'enduction, agent adhésif, agent nourrissant pour le cuir, agent humidifiant ou de traitement du textile, additifs pour matériaux de construction minéraux ou formulations cosmétiques, pharmaceutiques ou phytosanitaires, contenant des mélanges d'alcoxylates selon l'une quelconque des revendications 1 à 4.
